(19) Europäisches Patentamt
European Patent Office
Office européen des brevets

(11) EP 0 723 432 B1

(12) **EUROPÄISCHE PATENTSCHRIFT**

(45) Veröffentlichungstag und Bekanntmachung des Hinweises auf die Patenterteilung:
**30.12.1998 Patentblatt 1998/53**

(21) Anmeldenummer: **94928863.3**

(22) Anmeldetag: **05.10.1994**

(51) Int. Cl.$^6$: **A61K 7/00**

(86) Internationale Anmeldenummer:
**PCT/EP94/03293**

(87) Internationale Veröffentlichungsnummer:
**WO 95/10259 (20.04.1995 Gazette 1995/17)**

(54) **FLIESSFÄHIGES EMULSIONSKONZENTRAT**

FREE FLOWING EMULSION CONCENTRATE

CONCENTRE D'EMULSION COULANT

(84) Benannte Vertragsstaaten:
**AT BE DE ES FR GB IT**

(30) Priorität: **14.10.1993 DE 4335045**

(43) Veröffentlichungstag der Anmeldung:
**31.07.1996 Patentblatt 1996/31**

(73) Patentinhaber:
**Henkel Kommanditgesellschaft auf Aktien
40191 Düsseldorf (DE)**

(72) Erfinder:
- **WADLE, Armin**
  **D-40724 Hilden (DE)**
- **ANSMANN, Achim**
  **D-40699 Erkrath (DE)**
- **BAUMÖLLER, Guido**
  **D-40597 Düsseldorf (DE)**
- **TESMANN, Holger**
  **D-41363 Jüchen (DE)**

(56) Entgegenhaltungen:
**WO-A-92/07543         WO-A-93/11865
DE-A- 4 010 393**

**Beschreibung**

Die Erfindung betrifft ein bei Normaltemperatur fließ- und pumpfähiges Emulsionskonzentrat, dessen Verwendung Zur Herstellung von Öl-in-Wasser-Emulsionen sowie ein Verfahren zur Herstellung von Öl-in-Wasser-Emulsionen unter Verwendung des Emulsionskonzentrates.

Die Herstellung emulsionsförmiger Zubereitungen erfordert normalerweise einen erheblichen apparativen Aufwand, da die disperse Phase durch Erwärmen verflüssigt und unter Anwendung von Scherenergie in der kontinuierlichen Phase dispergiert werden muß. Es hat schon verschiedene Versuche gegeben, Emulsionskonzentrate herzustellen, die ohne Wärmezufuhr und ohne Scherarbeit mit der kontinuierlichen Phase verdünnt werden können.

So ist z.B. aus DE 23 05 087 C2 ein selbstemulgierendes Öl bekannt, das sich durch Zugabe von Wasser spontan und ohne Wärmezufuhr in eine Emulsion überführen läßt. Ein Nachteil solcher selbstemulgierenden Öle ist darin begründet, daß die damit herstellbaren Emulsionen nicht besonders feinteilig und daher nicht lange stabil sind. Sie lassen auch nicht die Einarbeitung weiterer Ölkomponenten oder höherschmelzende Wachse bei der Emulsionsherstellung zu.

Es bestand daher die Aufgabe, Emulsionskonzentrate zu entwickeln, die bei Normaltemperatur fließfähig und pumpbar sind und sich mit Wasser und Ölkomponenten und ggf. weiteren Zusätzen ohne Wärmezufuhr und unter Aufwendung nur geringer Rührenergie zu emulsionsförmigen Zubereitungen weiterverarbeiten lassen.

Überraschend wurde gefunden, daß diese Aufgabe durch hochkonzentrierte Emulsionen erfüllt wird, die hydrophile Emulgatoren und lipophile Coemulgatoren in definierten Mengenverhältnissen enthalten.

Gegenstand der Erfindung ist daher ein bei 20°C fließfähiges und pumpbares Emulsionskonzentrat mit einem Gehalt an wasserunlöslichen Ölkomponenten (A), hydrophilen, nicht-ionischen Emulgatoren (B), lipophilen Coemulgatoren (C) und Wasser, worin der Wassergehalt 50 - 70 Gew.-% des Konzentrats beträgt und die Komponenten (A), (B) und (C) im Gewichtsverhältnis A : B : C = 1 : (0,31 - 1,5) : (0,31 - 1,5) enthalten sind.

Als fließfähig oder pumpfähig werden dabei solche Emulsionskonzentrate bezeichnet, deren Viskosität bei 20°C unterhalb 20 Pa·s, gemessen mit einem Brookfield Rotationsviskosimeter (Typ RVF, Spindel TE, 4 Upm), liegt.

Als wasserunlösliche Ölkomponenten eignen sich alle bei 20°C flüssigen Fettstoffe oder Fettstoffgemische, d.h. auch Gemische aus flüssigen und darin gelösten, festen Fettstoffen oder Paraffinen, solange diese Gemische bei 20°C flüssig sind bzw. deren Viskosität (20°C) unter 20 Pa·s liegt.

Als flüssige Ölkomponenten sind vor allem die bei Raumtemperatur flüssigen Kohlenwasserstoffe und Fettsäureester geeignet. Flüssige Kohlenwasserstoffe sind z.B. Paraffinöle, flüssige Polyolefine oder Alkylcyclohexane, z.B. das 1,3-Diisooctylcyclohexan. Geeignete flüssige Fettsäureester sind z.B. die Methylester und Isopropylester von Fettsäuren mit 12 - 22 C-Atomen, z.B. Methyllaurat, Methylstearat, Methyloleat, Methylerucat, Isopropylpalmitat, Isopropylstearat, Isopropyloleat. Andere geeignete Ölkomponenten sind n-Butylstearat, n-Hexyllaurat, n-Decyloleat, Isooctylstearat, Isononylpalmitat, Isononyl-isononanoat, 2-Ethylhexyl-palmitat, 2-Ethylhexyl-laurat, 2-Hexyldecyl-stearat, 2-Octyl-dodecyl-palmitat, Oleyloleat, Oleylerucat, Erucyloleat sowie Ester, die aus technischen aliphatischen Alkoholgemischen und technischen, aliphatischen Carbonsäuren erhältlich sind, z.B. Ester aus gesättigten und ungesättigten Fettalkoholen mit 12 - 22 C-Atomen und gesättigten und ungesättigten Fettsäuren mit 12 - 22 C-Atomen, wie sie aus tierischen und pflanzlichen Fetten zugänglich sind. Geeignet sind auch natürlich vorkommende flüssige Wachsester, wie sie im Spermöl und Jojobaöl vorliegen.

Geeignete flüssige Dicarbonsäureester sind z.B. Di-n-butyl-adipat, Di-n-butyl-sebazat, Di-(2-ethylhexyl)-adipat, Di-(2-ethylhexyl)-succinat, Di-isotridecyl-azelaat. Geeignete Diolester sind z.B. Ethylenglykol-dioleat, Ethylenglykol-di-isotridecanoat, Propylenglykol-di-(2-ethylhexanoat), Butandiol-diisostearat oder Neopentylglykol-di-caprylat.

Auch flüssige Triglyceride wie z.B. Olivenöl, Sonnenblumenöl, Sojaöl, Erdnußöl, Rapsöl, Mandelöl, Palmöl oder die flüssigen Anteile des Kokos- oder Palmkernöls sowie tierische Öle wie z.B. Klauenöl, die flüssigen Anteile des Rindertalges, oder auch synthetische Triglyceridöle, wie sie z.B. durch Veresterung von Glycerin mit Caprylsäure-Caprinsäure-Gemischen, technischer Ölsäure oder Palmitinsäure-Ölsäure-Gemischen erhältlich, können als Ölkomponenten eingesetzt werden.

Bevorzugt eignen sich als Ölkomponenten (A) die bei 20°C flüssigen Kohlenwasserstoffe, Dialkylether und Fettsäureester mit jeweils 16 bis 36 C-Atomen oder Mischungen dieser Komponenten.

Als hydrophile nichtionogene Emulgatoren (B) eignen sich bevorzugt Anlagerungsprodukte von Ethylenoxid an lineare Fettalkohole, Fettsäuren, Fettsäurepartialglyceride, Sorbitan-Fettsäureester oder Alkyl-(oligo)-glycoside mit einem HLB-Wert von 11 - 19. Als HLB-Wert soll dabei der Wert gemäß Formel I

$$HLB = \frac{100 - L}{5} \qquad (I)$$

verstanden werden, in der L den Anteil (in Gew.-%) der lipophilen Alkyl- oder Acylgruppen in den Ethylenoxid-Anlagerungsprodukten bedeutet. Bevorzugt geeignete hydrophile nichtionogene Emulgatoren sind die Anlagerungsprodukte von 8 - 30 Mol Ethylenoxid an lineare Fettalkohole mit 12 - 22 C-Atomen.

Die lipophilen Coemulgatoren (C) sind nichtionogene, polare Lipidstoffe mit einer oder mehreren Hydroxylgruppen, die in Wasser unlöslich oder nur dispergierbar sind und die aufgrund ihrer niedrigen Hydrophilie allein nicht zur Herstellung von Öl-in-Wasser-Emulsionen geeignet sind.

Als Coemulgatoren sind erfindungsgemäß solche vom Typ der gesättigten Fettalkohole mit 16 - 22 C-Atomen, z.B. Cetylalkohol, Stearylalkohol, Arachidylalkohol oder Behenylalkohol oder Gemische dieser Alkohole geeignet, wie sie bei der technischen Hydrierung von pflanzlichen und tierischen Fettsäuren mit 16 - 22 C-Atomen oder der entsprechenden Fettsäuremethylester erhalten werden. Weiterhin eignen sich als Coemulgatoren Partialester aus einem Polyol mit 3 - 6 C-Atomen und gesättigten Fettsäuren mit 14 - 22 C-Atomen. Solche Partialester sind z.B. die Monoglyceride von Palmitin- und/oder Stearinsäure, die Sorbitanmono- und/oder -diester von Myristinsäure, Palmitinsäure, Stearinsäure oder von Mischungen dieser Fettsäuren, die Monoester aus Trimethylolpropan, Erythrit oder Pentaerythrit und gesättigten Fettsäuren mit 14 - 22 C-Atomen. Als Monoester werden auch die technischen Monoester verstanden, die durch Veresterung von 1 Mol Polyol mit 1 Mol Fettsäure erhalten werden und die ein Gemisch aus Monoester, Diester und unverestertem Polyol darstellen. Besonders gut eignen sich gesättigte Fettalkohole mit 16 - 22 C-Atomen oder Partialester von Polyolen mit 3 - 6 C-Atomen und Fettsäuren mit 14 - 22 C-Atomen.

Die Herstellung der erfindungsgemäßen Emulsionskonzentrate erfolgt bevorzugt nach dem in DE 38 19 193 beschriebenen Verfahren, es ist daher bevorzugt, die Ölkomponente (A), den hydrophilen Emulgator (B) und den lipophilen Coemulgator (C) so auszuwählen, daß die damit hergestellten Emulsionen eine Phaseninversionstemperatur im Bereich unter 100°C aufweisen. Die Emulgatoren (B) und Coemulgatoren (C) werden mit der Ölkomponente (A) gemischt und bis zur Phaseninversionstemperatur erwärmt. Dann wird Wasser von etwa gleicher Temperatur unter Rühren zugesetzt - oder umgekehrt, die Mischung von Ölkomponente, Emulgator und Coemulgator in das auf Phaseninversionstemperatur erwärmte Wasser eingerührt. Alternativ kann die Emulgierung auch unterhalb der Phaseninversionstemperatur durchgeführt und die Emulsion dann unter Wärmezufuhr kurzzeitig in den Phaseninversionstemperatur-Bereich gebracht werden. Nach dem Abkühlen erhält man dann ein sehr feinteiliges, bei 20°C noch fließfähiges Emulsionskonzentrat.

Das erfindungsgemäße Emulsionskonzentrat eignet sich insbesondere zur Herstellung kosmetischer und pharmazeutischer Öl-in-Wasser-Emulsionen. Dabei kann entweder die kontinuierliche wäßrige Phase oder die disperse Ölphase oder beides ohne weitere Wärmezufuhr in das Emulsionskonzentrat eingearbeitet werden.

Die wäßrige Phase, mit der das Emulsionskonzentrat verdünnt wird, kann beliebige wasserlösliche Inhaltsstoffe, z.B. wasserlösliche kosmetische Wirkstoffe, wasserlösliche Proteine oder Proteinabbauprodukte, Konservierungsmittel, Farbstoffe, Duftstoffe, Propylenglycol oder Glycerin, Magnesiumsalze oder andere übliche wasserlösliche Komponenten gelöst enthalten. Bevorzugt enthält die wäßrige, kontinuierliche Phase ein wasserlösliches, natürliches oder synthetisches Polymerisat, das die kosmetischen Eigenschaften der Emulsionen durch Erhöhung der Viskosität verbessert. Eine besonders wirksame Kombination von Hydrocolloiden zur Verbesserung der kosmetischen Eigenschaften solcher Emulsionen ist ein Gemisch aus nichtionischen Celluloseethern, z.B. Hydroxypropylcellulose und vernetzten Acrylsäure-Polymerisaten, wie sie z.B. unter der Handelsbezeichnung Carbopol[R] erhältlich sind (vgl. DE 35 21 713 A1).

Bevorzugt wird aber als Ölphase ein bei Normaltemperatur flüssiges Öl oder eine flüssige Mischung von Ölen und Fettkomponenten verwendet. Eine bevorzugte Ausführungsform der Erfindung ist daher ein Verfahren zur Herstellung von Öl-in-Wasser-Emulsionen, bei dem das erfindungsgemäße Emulsionskonzentrat ohne Wärmezufuhr mit einer bei 20°C flüssigen Ölkomponente mischt und mit Wasser oder einer wäßrigen Lösung eines wasserlöslichen Polymeren verdünnt.

Die erfindungsgemäßen Emulsionskonzentrate sind feinteilig und sehr lagerstabil, sie eignen sich daher ganz besonders als vorgefertigter Emulsionsbaustein, der aufgrund seiner Fließfähigkeit gut für die Lagerung und den Transport an einen Verarbeitungsort mit geringer technischer Ausrüstung geeignet ist, um dort mit einfachsten Mitteln brauchbare kosmetische und pharmazeutische Öl-in-Wasser-Emulsionen herzustellen.

Die folgenden Beispiele sollen die Erfindung näher erläutern:

**Beispiele**

**1. Emulsionskonzentrate**

| | 1 | 2 | 3 | V1 | V2 |
|---|---|---|---|---|---|
| 2-Ethylhexyl-stearat | 20 | 10 | - | 20 | 20 |

(fortgesetzt)

| | 1 | 2 | 3 | V1 | V2 |
|---|---|---|---|---|---|
| Cetyl-/Stearyl-isononanoat | - | - | 20 | - | - |
| Cetyl-palmitat | - | - | 0,8 | - | - |
| Cetyl-/Stearylalkohol + 12 Mol E0 | 7,5 | 1,8 | 0,8 | 5,6 | 4,0 |
| Cetyl-/Stearylalkohol + 20 Mol E0 | - | 7,6 | 9,0 | - | - |
| Cetyl-/Stearylalkohol (50 : 50) | 10 | 1,8 | 7,8 | 7,8 | 6,0 |
| Glycerin-mono/di-stearat | - | 9,1 | 3,8 | - | - |
| Wasser | 62,5 | 69,7 | 57,8 | 66,6 | 70 |
| Viskosität (23° C) Pa • s (Brookfield RVF, Spindel TE, 4 Upm) | 4 | 1,6 | 1,2 | 237 | 62 |

## 1.2 Herstellung

Die hydrophilen Emulgatoren und lipophilen Emulgatoren wurden zusammen mit der Ölkomponente bis zur Phaseninversionstemperatur (hier 85°C) erwärmt. Dann wurde das Wasser mit gleicher Temperatur (85°C) hinzugegeben und intensiv gemischt. Nach dem Abkühlen auf 20°C wurde eine feinteilige Emulsion erhalten. Die Viskositätsmessung erfolgte jeweils 5 Stunden nach Herstellung der Dispersion mit Hilfe eines Rotationsviskosimeters.

In der Vergleichsrezeptur V beträgt das Gewichtsverhältnis Öl (A) : Emulgator (B) : Coemulgator (C) = 1 : 0,28 : 0,39. Trotz dieser geringen Abweichung vom erfindungsgemäßen Mengenverhältnis ist die Emulsion nicht mehr fließfähig.

## 2. Anwendungsbeispiel

### 2.1 Rezeptur

| Emulsionskonzentrat gemäß Beispiel 1 | 20 Gew.-% |
|---|---|
| Paraffinöl | 2 Gew.-% |
| Capryl-/Caprinsäure-triglycerid | 4 Gew.-% |
| Vernetzte Polyacrylsäure (2 %ige Dispersion) | 20 Gew.-% |
| Natronlauge (1 %ig in Wasser) | 10 Gew.-% |
| Glycerin (86 %ig) | 5 Gew.-% |
| Wasser | 39 Gew.-% |

### 2.2 Herstellung

Die Ölkomponenten wurden bei 20°C mit dem Emulsionskonzentrat vermischt und unter Rühren emulgiert. Dann wurden die Polymerquellung (Polyacrylsäure-Disperson, NaOH), Glycerin und Wasser eingemischt.

## Patentansprüche

1. Bei 20°C fließfähiges und pumpbares Emulsionskonzentrat mit einem Gehalt an wasserunlöslichen Ölkomponenten (A), hydrophilen nichtionischen Emulgatoren (B), lipophilen Coemulgatoren (C) und Wasser, dadurch gekennzeichnet, daß der Wassergehalt 50 - 70 Gew.-% des Konzentrats beträgt und die Komponenten (A), (B) und (C) im Gewichtsverhältnis A : B : C = 1 : (0,31 - 1,5) : (0,31 - 1,5) enthalten sind.

2. Emulsionskonzentrat nach Anspruch 1, dadurch gekennzeichnet, daß die wasserunlösliche Ölkomponente (A) ausgewählt ist aus bei 20°C flüssigen Kohlenwasserstoffen, Dialkylethern und Fettsäureestern mit 16 - 36 C-Atomen oder deren Mischungen.

3. Emulsionskonzentrat nach Anspruch 1 oder 2, dadurch gekennzeichnet, daß als hydrophile Emulgatoren (B) Anlagerungsprodukte von Ethylenoxid an lineare Fettalkohole, Fettsäuren, Fettsäurepartialglyceride, Sorbitan-Fettsäureester oder Alkylglycoside mit einem HLB-Wert von 11 - 19 enthalten sind.

4. Emulsionskonzentrat nach Anspruch 1 - 3, dadurch gekennzeichnet, daß als lipophile Coemulgatoren (C) gesättigte Fettalkohole mit 16 - 22 C-Atomen oder Partialester von Polyolen mit 3 - 6 C-Atomen und Fettsäuren mit 14 - 22 C-Atomen oder Gemischen davon enthalten sind.

5. Verwendung der Emulsionskonzentrate gemäß Anspruch 1 - 4 zur Herstellung kosmetischer oder pharmazeutischer Öl-in-Wasser-Emulsionen.

6. Verfahren zur Herstellung von Öl-in-Wasser-Emulsionen, dadurch gekennzeichnet, daß man ein Emulsionskonzentrat gemäß Anspruch 1 bis 4 ohne Wärmezufuhr mit bei 20°C flüssigen Ölkomponenten mischt und mit Wasser und/oder einer wäßrigen Lösung eines wasserlöslichen Polymeren verdünnt.

## Claims

1. An emulsion concentrate which is flowable and pumpable at 20°C and which contains water-insoluble oil components (A), hydrophilic nonionic emulsifiers (B), lipophilic co-emulsifiers (C) and water, characterized in that the water content is from 50 to 70% by weight, based on the concentrate, and components (A), (B) and (C) are present in a ratio by weight of A to B to C of 1 : (0.31 - 1.5) : (0.31 - 1.5).

2. A emulsion concentrate as claimed in claim 1, characterized in that the water-insoluble oil component (A) is selected from hydrocarbons, dialkyl ethers and fatty acid esters containing 16 to 36 carbon atoms which are liquid at 20°C or mixtures thereof.

3. A emulsion concentrate as claimed in claim 1 or 2, characterized in that adducts of ethylene oxide with linear fatty alcohols, fatty acids, fatty acid partial glycerides, sorbitan fatty acid esters or alkyl glycosides with an HLB value of 11 to 19 are present as the hydrophilic emulsifiers (B).

4. An emulsion concentrate as claimed in claims 1 to 3, characterized in that saturated fatty alcohols containing 16 to 22 carbon atoms or partial esters of polyols containing 3 to 6 carbon atoms and fatty acids containing 14 to 22 carbon atoms or mixtures thereof are present as the lipophilic co-emulsifiers (C).

5. The use of the emulsion concentrates claimed in claims 1 to 4 for the production of cosmetic or pharmaceutical oil-in-water emulsions.

6. A process for the production of oil-in-water emulsions, characterized in that an emulsion concentrate according to claims 1 to 4 is mixed with oil components liquid at 20°C without heating and the resulting mixture is diluted with water and/or an aqueous solution of a water-soluble polymer.

## Revendications

1. Concentré d'émulsion coulant et pompable à 20 °C, renfermant des composants huileux insolubles dans l'eau (A), des émulsionnants non ioniques hydrophiles (B), des coémulsionnants lipophiles (C) et de l'eau, caractérisé en ce que la teneur en eau atteint 50 à 70 % en poids du concentré et les composés (A), (B) et (C) sont contenus dans un rapport pondéral A : B : C égal à 1 : (0,31 - 1,5) : (0,31 - 1,5).

2. Concentré d'émulsion selon la revendication 1, caractérisé en ce que le composant huileux insoluble dans l'eau (A) est sélectionné parmi les hydrocarbures, les dialkyléthers et les esters d'acides gras comportant 16 à 36 atomes de carbone et liquides à 20 °C ou les mélanges de ceux-ci.

3. Concentré d'émulsion selon la revendication 1 ou 2, caractérisé en ce qu'il renferme comme émulsionnants hydrophiles (B), des produits d'addition d'oxyde d'éthylène à des alcools gras linéaires, des acides gras, des glycérides partiels d'acides gras, des esters d'acides gras de sorbitane ou des alkylglycosides possédant une valeur HLB de 11 à 19.

4. Concentré d'émulsion selon les revendications 1 à 3, caractérisé en ce qu'il renferme comme coémulsionnants lipophiles (C), des alcools gras saturés comportant 16 à 22 atomes de C, des esters partiels de polyols comportant 3 à 6 atomes de C et d'acides gras possédant 14 à 22 atomes de C ou des mélanges de ceux-ci.

5. Utilisation des concentrés d'émulsion selon les revendications 1 à 4 pour la confection d'émulsions huile dans eau cosmétiques ou pharmaceutiques.

6. Procédé de confection d'émulsions huile dans eau, caractérisé en ce que l'on mélange un concentré d'émulsion selon les revendications 1 à 4, sans apport de chaleur, avec des composants huileux liquides à 20 °C et que l'on dilue ce mélange avec de l'eau et/ou avec une solution aqueuse d'un polymère soluble dans l'eau.